# FASCICULE DE BREVET EUROPEEN

(11) **EP 3 145 588 B1**
(45) Date de publication et mention de la délivrance du brevet: **10.03.2021**
(21) Numéro de dépôt: 15732791.7
(22) Date de dépôt: 22.05.2015
(51) Int. Cl.: A61Q 1/02, A61Q 1/06, A61K 8/02, A45D 40/16

(54) **PRODUITS COSMÉTIQUES MULTI-COMPOSITIONS ET LEUR PROCÉDÉ DE FABRICATION**
KOSMETISCHE PRODUKTE MIT MEHREREN ZUSAMMENSETZUNGEN UND VERFAHREN ZUR HERSTELLUNG DAVON
MULTI-COMPOSITION COSMETIC PRODUCTS AND METHOD FOR MANUFACTURING SAME

(30) Priorité: 23.05.2014 FR 1454677
(43) Date de publication de la demande: 29.03.2017
(73) Titulaire: LVMH RECHERCHE, 45800 St. Jean de Braye (FR)
(72) Inventeur: PERRIN, Lily-Ann, F-45190 Beaugency (FR); DE LA POTERIE, Valérie, F-45740 Lailly en Val (FR)
(74) Mandataire: Cabinet Beau de Loménie
(86) Numéro de dépôt international: PCT/FR2015/051368
(87) Numéro de publication internationale: WO 2015/177484

(56) Documents cités:
- WO-A1-01/91605
- WO-A2-02/072044
- FR-A- 1 396 720
- US-A- 4 602 886
- US-A1- 2007 059 263

## Description

L'invention concerne un nouveau procédé de fabrication de produits cosmétiques comprenant au moins une composition cosmétique, solide à température ambiante, contenant une huile, laquelle composition est destinée à être appliquée sur une partie du corps pour le soin, le maquillage ou le parfumage de la peau ou des cheveux. Les produits cosmétiques obtenus selon le procédé de l'invention présentent de nouvelles formes, de nouvelles associations de teintes, ainsi que de nouvelles associations de textures et de matières. Les produits cosmétiques obtenus présentent également des formes en relief très originales qu'il était impossible de réaliser avec les techniques actuelles.

Le procédé de l'invention consiste à faire fondre la composition pour la fluidifier, puis à couler cette composition à l'état fluide sur une surface par dépôts successifs de très petite taille qui peuvent prendre la forme de feuilles, de fils ou de gouttes. Le procédé de fabrication de l'invention ne nécessite pas l'utilisation d'un moule, qui était jusqu'à présent nécessaire pour fabriquer des produits cosmétiques coulés.

Grâce au procédé de l'invention, on peut facilement concevoir des produits cosmétiques multi-compositions, c'est-à-dire constitués de plusieurs compositions solides différentes, en surface ou dans la masse, visibles ou cachées. Ce procédé permet également de créer des reliefs très fins en surface de ces produits.

### ART ANTERIEUR

Les compositions cosmétiques solides qui contiennent des corps gras en fortes proportions sont généralement fabriquées par coulage à chaud, soit dans un moule qui leur donne une forme, soit dans une coupelle ou un godet.

Ainsi, les articles de maquillage qui offrent au consommateur une surface plane pour prélever le produit, par exemple les fards à joues, les fonds de teint ou les fards à paupières, comportent un boîtier avec un couvercle et un réceptacle. Ces produits sont obtenus par coulage à chaud du produit liquéfié dans la coupelle par le dessus ou par le dessous via des trous percées dans la coupelle. Celle-ci est ensuite placée dans le boîtier.

Les produits de maquillage en relief, comme les sticks, sont fabriqués par moulage. La composition est fondue au-dessus de sa température de fusion, puis coulée dans le moule, mise à refroidir et démoulée. Le produit démoulé est ensuite placé dans un récipient doté ou non d'un mécanisme tournant pour son extraction ou sa manutention.

Les moules sont en métal ou en matière plastique, par exemple en silicone ou en élastomère SEBS, pour un démoulage des objets plus facile. Les moules métalliques manuels sont réalisés en plusieurs parties qui sont écartées pour permettre le démoulage. Les moules métalliques industriels sont réalisés en une partie qui dispose d'une fente par lequel de l'air comprimé éjecte le stick au moment du démoulage. Ces moules métalliques nécessitent un traitement de surface tell qu'une pulvérisation d'une huile de silicone sur les parois pour faciliter le démoulage et améliorer l'état de surface des objets démoulés.

Le produit cosmétique est fondu au-delà de sa température de fusion puis versé dans les empreintes des moules qui peuvent être préalablement préchauffés à une température de l'ordre de 40°C pour éviter une prise en masse trop rapide. Les moules sont refroidis, une fois remplis, par contact avec une table réfrigérante ou dans une enceinte réfrigérée dont la température est comprise entre - 20°C et 15°C.

Les procédés de moulage des sticks présentent donc de nombreux inconvénients, tels que le prix élevé de conception et de fabrication des moules de haute précision, la nécessité d'atteindre des températures inférieures à 0°C pour permettre le démoulage, des durées importantes de refroidissement des produits qui ont été coulés dans les moules, l'obligation de nettoyer les moules après chaque usage, et la nécessité de chauffer à nouveau les moules pour effectuer un nouveau cycle de fabrication. L'exploitation de ce type de procédé comportent d'autres inconvénients tels que la révision fréquente des moules métalliques ou le remplacement régulier des moules silicone, révision rendue nécessaire par les chocs mécaniques et thermiques répétés ou encore l'imprégnation d'ingrédients des compositions cosmétiques dans la matière des moules silicone Les sticks de rouge à lèvres peuvent présenter des variantes de relief et de teintes, mais les reliefs sont limités à quelques dixièmes de millimètres et le nombre de teintes dépasse rarement deux ou trois. Des effets de teinte ont été proposés mais ils sont toujours géométriques : de type radial (double cylindre de structure cœur-écorce) ou linéaire (des couches verticales ou horizontales). Enfin, des motifs de teintes aléatoires ont déjà été proposés en coulant deux teintes à l'état fluide en même temps dans le moule.

Les structures de sticks cœur/écorce sont obtenues par moulage. On fabrique par exemple un rouge à lèvres multicolores en utilisant une paire de moules : un moule noyau placé au centre d'un moule cylindrique. On réalise une première coulée d'une première teinte dans la partie creuse du moule noyau, que l'on laisse refroidir et solidifier. Après avoir enlevé le moule central, la cavité laissée après l'enlèvement du noyau est remplie avec une deuxième teinte pour obtenir le rouge à lèvres multicolore. Comme méthode alternative, une plaque de séparation peut être insérée pour créer des cavités semi-cylindriques, puis être retirée après solidification des différentes teintes. Cette méthode nécessite cependant que les teintes soient ensuite rendues solidaires par chauffage modéré de leur surface pour permettre leur assemblage.

Les structures de sticks côte à côte sont préparées avec un premier moule ayant une cavité hémi-cylindrique (comprenant deux pièces dont une plaque plane verticale amovible). Une première masse fondue est versée dans cette cavité et refroidie pour solidifier la composition. La plaque est retirée puis remplacée par un deuxième moule identique au premier que l'on place en miroir et au contact de la première composition. Une deuxième masse fondue est versée dans la cavité et refroidie. Après démoulage, les deux compositions hémicylindriques reliées ensemble selon un plan radial.

Des produits de maquillage comprenant un ou deux motifs en relief de couleurs différentes affleurant d'une surface plane ont été proposés par moulage dans le brevet FR 2956833, en utilisant deux moules superposés. Une coupelle rigide à fond plat percée d'un orifice est positionnée sur un moule déformable. Le produit de maquillage sous forme liquide est versé dans le moule déformable par l'orifice de la coupelle, de sorte que le produit démoulé obtenu comporte un ou plusieurs motifs sur la surface libre proposée au consommateur. Les reliefs obtenus ont une hauteur de l'ordre de 1 à 2 mm.

Le document WO 01/91605 décrit un produit cosmétique, tel qu'un baume à lèvres ou un déodorant cœur/écorce, en forme de bâton comprenant plusieurs compositions qui diffèrent par leur couleur ou leur parfum par exemple. Le produit comprend un mécanisme à vis permettant de sortir ou de rentrer le bâton dans un récipient cylindrique. Le procédé de fabrication d'un tel produit comprend les étapes consistant à insérer un moule creux dans le récipient, à distribuer une première composition dans l'espace situé entre la paroi intérieure du récipient et la paroi extérieure du moule, à retirer le moule du récipient pour former une cavité, puis à remplir la cavité avec une deuxième composition.

Dans le document US 4 602 886, on fabrique un rouge à lèvres par coulages successifs de petites quantités de matériaux fondus dans un moule cylindrique. Le produit, en forme de bâton, comporte une multiplicité de dépôts de couleurs différentes et de forme aléatoire, répartis de manière aléatoire. Le récipient du rouge à lèvres sert de moule pendant le coulage des matériaux fondus.

Les inventeurs ont également tenté d'effectuer plusieurs coulées dans un moule pour créer un stick de rouge à lèvres constitué de plusieurs couches horizontales de différentes couleurs. Mais ce procédé reste très limité, car l'épaisseur minimale des couches doit être importante pour garantir leur régularité, typiquement de l'ordre de quelques millimètres.

Tous ces procédés présentent cependant le désavantage de nécessiter plusieurs moules, et plusieurs étapes de coulage espacées dans le temps, car il est nécessaire d'attendre le refroidissement et la solidification de la première teinte avant de couler la deuxième pour éviter leur mélange.

Au surplus, dans tous les produits multicolores coulés de l'art antérieur, les couches de produits ont nécessairement une surface plane du fait de la gravité : soit parallèle à l'axe vertical du stick, soit perpendiculaire à l'axe vertical du stick, ce qui limite considérablement les possibilités de présentation des produits que l'on souhaite multi-teintes.

Enfin, les moules sont coûteux à fabriquer, surtout lorsque l'on souhaite réaliser des formes irrégulières, et nécessitent une inspection et un entretien fréquents.

La grande majorité des rouges à lèvres commercialisés se présentent sous forme de sticks ou sont coulés en godets. La géométrie de ces produits reste très symétrique et basique. Bien que les rouges à lèvres aient des aspects, des textures, des teintes et des propriétés galéniques variées, aucune de ces spécificités ne se retrouvent généralement combinées dans un seul et même produit.

Le besoin subsiste donc de proposer des produits cosmétiques multi-compositions proposant de nouveaux arrangements de teinte, de texture ou de matière plus faciles, plus rapides et moins onéreux à fabriquer. On recherche également à fabriquer des produits cosmétiques au relief plus irrégulier et plus fin.

### OBJECTIFS DE l'INVENTION

On souhaite développer de nouvelles formes et de nouvelles présentations de compositions cosmétiques solides contenant une huile, notamment en forte proportion, en particulier dans le domaine du maquillage.

Dans le cadre de l'invention, les inventeurs ont trouvé qu'il est possible de façonner de telles compositions selon des formes, des reliefs et des teintes très variées pour former des produits multi-teintes. Ils ont également trouvé un procédé permettant de couler au cours d'une même étape des compositions cosmétiques de texture différente pour former des produits multi-textures. Enfin, ils ont trouvé un procédé permettant de couler de façon simultanée une composition cosmétique et un matériau support non cosmétique thermoplastique, pour former des produits multi-matières.

Les inventeurs ont ainsi trouvé un nouveau procédé de coulage à chaud d'une composition solide qui ne nécessite pas l'usage d'un moule ou d'un récipient comme c'est le cas dans tous les procédés de l'art antérieur. Ce procédé permet notamment d'obtenir des motifs très fins, des entrelacements de rubans, ainsi que des dégradés de couleur, ce qui n'avait jamais été réalisé auparavant.

Le procédé de l'invention présente de multiples avantages. Il permet de façonner des produits dont la surface ou la masse comporte une pluralité de formes et de domaines de couleurs différentes. La surface de ces formes et de ces domaines n'est pas nécessairement plane.

La présente invention concerne donc un nouveau procédé pour fabriquer un produit cosmétique solide, qui consiste à faire fondre une composition, à la couler à l'état fluide sur un support selon un motif prédéfini en réalisant une pluralité de dépôts successifs ou simultanés, puis à les laisser refroidir.

### DEFINITIONS

Au sens de la présente invention, on entend par « composition cosmétique », le mélange de plusieurs ingrédients dont une huile formant un objet solide à 25°C. La composition cosmétique est destinée à être appliquée telle qu'elle, sur une partie du corps pour le soin, le maquillage ou le parfumage de la peau ou des cheveux.

Par « produit cosmétique », un produit destiné à la vente qui comprend au moins une composition cosmétique éventuellement associée à des moyens d'application et présentée dans un récipient.

Par « produit cosmétique multi-composition», on entend un produit cosmétique comprenant au moins deux, de préférence au moins trois compositions solides, différentes dans les ingrédients qui les composent. La totalité des compositions peuvent apparaître différentes à la surface du produit et être visibles par l'utilisateur. Alternativement, une première composition peut être située dans la masse du produit, si bien que l'utilisateur peut la percevoir par transparence d'une deuxième composition qui la masque, ou la découvrir une fois que la deuxième composition qui la masque a été utilisée par applications successives sur le corps, le visage ou les cheveux.

Par « produit multi-teinte », on entend un produit cosmétique comprenant au moins deux, de préférence au moins trois ou quatre compositions cosmétiques de teintes différentes.

Par « produit multi-texture », on entend un produit cosmétique comprenant au moins deux compositions cosmétiques ayant des textures différentes, c'est-à-dire provoquant un toucher à la prise ou à l'application différent selon leur consistance et leur propriétés rhéologiques. Comme exemple de textures, on peut citer les émulsions eau-dans-huile, les gels et les produits anhydres.

Par « produit multi-matière », on entend un produit comprenant au moins une composition cosmétique et au moins un matériau support non cosmétique thermoplastique, lequel matériau support a été mis en forme selon le procédé de l'invention.

On entend par "solide" une composition présentant, à 25°C et à pression atmosphérique (1 atm = 10⁵ Pa), une dureté supérieure à 0,5 N, de préférence supérieure à 2 N, ladite dureté étant de préférence mesurée à l'aide d'un texturomètre muni d'une sonde hémisphérique de diamètre 12,7 mm.

Par « solidifié », on entend partiellement ou totalement solidifié, c'est-à-dire qu'une partie de la masse ou la totalité de la masse du dépôt est à une température inférieure à son point de fluidification.

Par « fil », on entend un volume, par exemple essentiellement cylindrique, dont le diamètre moyen est très inférieur à sa longueur. Le diamètre est défini comme la plus grande dimension mesurée dans un plan perpendiculaire à la longueur. Le fil peut avoir un diamètre moyen compris entre 0,01 mm à 5 mm, par exemple entre 0,1 mm et 5 mm, de préférence entre 0,2 et 2,5 mm, de préférence encore entre 0,2 et 1,2 mm. Dans un mode de réalisation, le fil a un diamètre moyen de l'ordre de 0,5 mm. La section du fil peut être circulaire, ovale ou essentiellement rectangulaire. Lorsque la section est essentiellement rectangulaire, les dimensions du fil peuvent être définies par sa hauteur et sa largeur, laquelle correspond au diamètre moyen défini plus haut.

Par « goutte », on entend un volume essentiellement sphérique de diamètre moyen compris entre 0,01 et 5 mm, par exemple entre 0,1 mm et 5 mm, de préférence entre 0,1 et 1 mm.

Par « feuille », on entend un volume essentiellement parallélépipédique dont la largeur est très supérieure à son épaisseur.

Par « point de fluidification », on entend la température à laquelle au moins une partie de la composition solide à 25°C passe à l'état liquide. Le point de fluidification de la composition peut être la température de fusion T50 à laquelle 50% de la composition est liquide, la température de fusion T80 à laquelle 80% de la composition est liquide, la température de fusion T90 à laquelle 90% de la composition est liquide, le point de goutte de la composition ou la température de transition sol-gel de la composition.

Par « huile », on entend un corps gras liquide à 15°C. Par "cire", au sens de la présente invention, entend désigner un composé solide à 25 °C qui présente un changement d'état solide/liquide réversible et une température de fusion supérieure à 30 °C. Par "composé gras pâteux", on désigne un composé gras non cristallin comprenant à une température de 25°C, une fraction liquide et une fraction solide.

Dans la présente demande, l'expression exprimant une plage de valeurs « aller de...à » comprend les bornes, tandis que l'expression exprimant une plage de valeurs « compris entre ...et » exclut les bornes.

### DESCRIPTION DE l'INVENTION

L'invention porte sur un procédé de fabrication d'un produit cosmétique par coulage, lequel procédé comprend la succession d'étapes suivantes :
a) la fluidification d'au moins une composition contenant une huile, dotée d'un point de fluidification compris entre 30°C et 150°C, et d'une dureté moyenne - mesurée à 25°C à l'aide d'un texturomètre muni d'une sonde hémisphérique - comprise entre 0,5 N et 80 N, en la chauffant à une température de fluidification supérieure à son point de fluidification,
b) l'introduction de la composition fluidifiée dans au moins une buse de coulage,
c1) le coulage par éjection de la composition fluidifiée à travers un orifice de la buse pour obtenir un volume, lequel volume est déposé sur un support et forme après refroidissement à 25°C un premier dépôt de ladite composition sur le support,
c2) la mise en mouvement relative - dans au moins une des trois directions de l'espace - de la buse par rapport audit support, le coulage de la composition fluidifiée à l'étape c1) et la mise en mouvement relative de la buse à l'étape c2) étant concomitants, de manière à former un premier dépôt de forme cylindrique, et la forme du volume éjecté étant identique à la forme du dépôt solide, en l'absence de contrainte mécanique ou thermique appliquée sur le dépôt après coulage,
d) l'arrêt du coulage, puis éventuellement
e) le déplacement relatif de la buse par rapport au support, et
f) la répétition des étapes a) à e) au moins une fois pour former au moins un autre dépôt sur ledit support, en utilisant la même composition ou une autre composition qui contient une huile, qui est dotée d'un point de fluidification compris entre 30 °C et 150°C, et qui a une dureté moyenne - mesurée à 25°C à l'aide d'un texturomètre muni d'une sonde hémisphérique - comprise entre 0,5 N et 80 N.

Dans ce procédé, le dépôt présente avantageusement la forme de gouttes ou des fils.

On peut préparer avant le démarrage du procédé de l'invention, dans une cuve de fluidification, un volume de composition fluidifiée correspondant au volume de tous les dépôts de composition qui seront nécessaire pour fabriquer le produit cosmétique dans son ensemble. On peut au contraire décider de fluidifier au fur et à mesure la quantité de composition correspondant à un seul.

Selon un de ses aspects, l'invention concerne un procédé de fabrication d'un produit cosmétique par coulage, sans usage d'un moule

Le volume de composition une fois déposé sur le support forme un dépôt qui se solidifie partiellement ou totalement. Le volume éjecté, le débit de coulage, la température de coulage et la température du support peuvent être choisis de manière à ce que le dépôt ait essentiellement la même forme que le volume. On souhaite de préférence que la forme du volume éjecté soit identique à la forme du dépôt solide, en l'absence de contrainte mécanique ou thermique appliquée sur le dépôt après coulage.

Le dépôt pourra avoir une forme différente du volume éjecté, notamment par application d'une contrainte thermique, par exemple par chauffage du support à une température supérieure à 25°C. Le dépôt pourra avoir une forme différente du volume éjecté, notamment par application d'une contrainte mécanique, par exemple en déposant un deuxième dépôt sur un premier dépôt, avant solidification du premier, en appliquant une force de pression visant à déformer le premier dépôt. On préfère que le dépôt conserve la forme du volume obtenu en sortie de la buse. Dans le procédé de l'invention, le support ne sert pas de contenant au volume de composition fluidifiée pendant son refroidissement, si bien que le dépôt solide tient sa forme essentiellement de l'orifice de la buse, en l'absence de toute contrainte mécanique ou thermique appliquée postérieurement au coulage.

Dans un mode de réalisation, la forme du volume éjecté est identique à la forme du dépôt solide, notamment en l'absence de contrainte mécanique ou thermique appliquée sur le dépôt après coulage. Ainsi, le diamètre moyen du dépôt peut être égal au diamètre de l'orifice de la buse.

On définira la température de coulage comme la température de l'espace défini entre la sortie de la buse et le support. Cet espace pourra être une enceinte thermostatée. La température de coulage est de préférence inférieure ou égale à la température de fluidification.

Le procédé de l'invention présente l'avantage de pouvoir concevoir de nouveaux produits cosmétiques dans des temps de développement plus courts. En effet, le débit de composition coulée peut être avantageusement inférieur à celui des procédés de coulage à chaud de l'art antérieur, de manière à supprimer l'étape de refroidissement nécessaire entre deux étapes de coulage de deux compositions différentes. Le coulage du procédé de l'invention peut être réalisé en régime quasi continu.

Une fois le dépôt de la composition coulé sur le support, celui-ci possède une surface en contact avec le support, une surface libre et éventuellement une surface en contact avec un dépôt qui a été coulé précédemment. Le procédé de coulage de l'invention permet avantageusement de créer un dépôt dont l'une de ses surfaces est non plane. La surface libre du dépôt est de préférence non plane.

Lorsque le support définit un volume creux, les dépôts effectués selon le procédé de l'invention présentent l'avantage de ne pas remplir ce volume, contrairement aux procédés de coulage de l'art antérieur qui utilise un moule comme support.

Le dépôt de composition une fois solidifié présente la particularité de posséder au moins une de ses trois dimensions inférieure à 5 mm, de préférence comprise entre 0,01 mm et 3 mm. Aussi, le dépôt peut être sous la forme d'un cylindre (ou fil), d'une sphère (ou goutte) ou d'une feuille à la sortie de la buse. La longueur d'un dépôt coulé selon le procédé de l'invention lorsqu'il est sous la forme d'un fil peut être supérieure à 1 cm, par exemple comprise entre 4 cm et 2 m, pour un diamètre moyen de 1 mm.

L'étape a) du procédé de l'invention consiste en la fluidification de la composition en la chauffant à une température de fluidification supérieure à son point de fluidification.

Au cours de l'étape a), la composition est rendue fluide par apport de chaleur, en partant de son état qui est solide à 25°C.

Selon un mode de mise en œuvre, dans une étape préalable à l'étape a), les ingrédients servant à la préparation de la composition dont l'huile sont mélangés à chaud, puis le mélange obtenu est refroidi, et éventuellement stocké en vue de son utilisation dans l'étape a).

On peut également préparer dans une première étape préalable à l'étape a) une base constituée de tous les ingrédients servant à la préparation de la composition à l'exception des matières colorantes telles que les pigments, les nacres et les colorants, puis dans une deuxième étape préalable à l'étape a), ajouter les matières colorantes qui auront été choisies par le consommateur.

La masse fluide résultant de l'une de ces deux variantes est de préférence maintenue sous agitation en continu, dans un mélangeur, à une température constante, de préférence à la température de fluidification, qui est supérieure au point de fluidification de la composition.

La composition peut être mélangée par exemple dans une extrudeuse à vis, avant d'être introduite dans la buse de coulage. Selon une variante, la composition peut être introduite sous forme d'une poudre solide dans une extrudeuse à vis dotée d'un moyen de chauffage qui porte la composition à la température de fluidification pour provoquer sa fluidification. L'extrémité de l'extrudeuse délivrant la masse fluide de composition est avantageusement reliée à un moyen d'alimentation de la buse de coulage.

La composition est avantageusement adaptée pour que - à la température de coulage - sa viscosité permette son écoulement sous la forme d'un dépôt régulier sur le support, et pour que le dépôt formé se solidifie selon la cinétique souhaitée en fonction du résultat attendu. On peut souhaiter que le dépôt se solidifie très rapidement ou au contraire lentement pour faciliter son adhésion à un dépôt ultérieur. La vitesse de solidification de la surface libre peut être modulée par thermostatage de l'air ambiant. Dans un mode de mise en œuvre, la solidification de la surface libre des dépôts s'effectue avantageusement en quelques secondes à température ambiante (25°C). Le dépôt peut présenter l'avantage de se solidifier en quelques secondes, si bien qu'il n'est pas nécessaire de marquer un arrêt entre deux séries d'étapes a) à e).

Le point de fluidification de la composition est de préférence compris entre 30°C et 100°C, de préférence entre 40°C et 95°C.

Selon la nature de la composition et des composés utilisés pour structurer l'huile qu'elle renferme, on peut définir le point de fluidification de la composition comme étant la température de fusion à laquelle une partie ou la totalité de sa masse est à l'état liquide. Par exemple la température TX est la température à laquelle X% de la composition est à l'état liquide, X étant un nombre compris entre 1 et 100. On préfère utiliser dans le cadre de la présente invention les paramètres T50, T90 et T95, la température T50 étant la température à laquelle 50% de la composition est à l'état liquide, la température T90 étant la température à laquelle 90% de la composition est à l'état liquide et la température T95 est la température à laquelle 95% de la composition est à l'état liquide. Ces températures peuvent être mesurées à l'aide d'un calorimètre à balayage différentiel (DSC), tel que le calorimètre vendu sous la dénomination DSC 30 par la société Mettler, avec une montée en température de 5 ou 10 °C par minute. La courbe représentant l'énergie absorbée par l'échantillon pour fondre en fonction de la température (thermogramme) définit une aire. TX représente la température à laquelle la valeur de l'aire sous la courbe à TX est égale à X% de l'aire totale sous la courbe.

L'enthalpie de fusion totale de la composition est égale à l'aire sous la courbe de son thermogramme obtenu à l'aide d'un calorimètre à balayage différentiel tel que le calorimètre vendu sous la dénomination MDSC 2920 par la société TA instrument, avec une montée en température de 5 ou 10°C par minute. L'enthalpie de fusion de la composition exprimée en J/g représente la quantité d'énergie nécessaire pour faire passer la composition de l'état solide à l'état liquide.

Ainsi, l'enthalpie de fusion consommée à X°C est la quantité d'énergie absorbée par l'échantillon pour passer de l'état solide à l'état qu'il présente à X°C constitué d'une fraction liquide et d'une fraction solide.

La fraction liquide de la composition mesurée à X°C est égale au rapport de l'enthalpie de fusion consommée à X°C et de l'enthalpie de fusion totale de la composition.

On peut également définir le point de fluidification comme étant le point de goutte de la composition. Le point de goutte peut être mesuré avec un appareil doté de cupules normalisées de diamètre d'orifice 2,8 mm, en adoptant une vitesse de chauffe de 1,0°C/min ou 2,0°C/min, une température de départ de 20°C inférieure au point de goutte attendu, et une température de fin de 10°C à 15°C supérieure ou point de goutte attendu. La goutte peut être détectée visuellement à l'aide d'une caméra intégrée à l'appareil ou par une cellule de mesure. L'appareil retient la température à laquelle la goutte a coulé dans un manchon collecteur.

L'appareil peut être le modèle Mettler Thermosystème FP900 associé à une cellule de mesure Mettler FP83HT, ou le modèle Toledo DP70. La composition est coulée à chaud dans la cupule, puis mise à refroidir et maintenue dans une enceinte à 20°C pendant 4 heures.

On peut également définir le point de fluidification comme étant la température de transition sol-gel de la composition telle que définie par exemple dans le document Polymer Journal, 18 (5), 411-416 (1986).

On peut encore définir le point de fluidification comme étant le point de ramollissement de la composition. Par exemple, le point de ramollissement mesuré selon cette méthode peut être compris entre 55°C à 85°C.

Pour mesurer le point de ramollissement de la composition par la méthode de la bille et de l'anneau, un anneau en laiton de dimensions définies est rempli de composition fondue, et placé sur son support. Une bille en acier (diamètre=9,53 mm, masse=3,5 g) est sur la composition au centre de l'anneau. Le support portant l'anneau rempli est ensuite immergé dans un bécher (au moins 85 mm de diamètre intérieur et plus de 120 mm de hauteur) contenant de l'eau déminéralisée, du glycérol ou une huile silicone, selon la gamme de températures que l'on souhaite balayer. La température du bain est augmentée de 5°C.min⁻¹. On note la température à laquelle la matière entourant la bille se détache de l'anneau et tombe sur la plaque inférieure du support.

La composition utilisée dans le cadre de l'invention est de préférence dotée d'un point de goutte supérieur à 50°C, par exemple compris entre 70°C et 85°C. Le point de goutte de la composition peut être supérieur à 50°C, de préférence supérieur à 55°C, par exemple compris entre 55°C et 60°C, compris entre 70°C et 80°C, ou entre 75°C et 85°C.

La composition peut être dotée d'une température T50 comprise entre 40°C et 70°C, par exemple entre 55°C et 60°C. La composition peut répondre à un T90 compris entre 70°C et 85°C, plus précisément entre 75°C et 80°C.

La température de fluidification est de préférence supérieure de 1°C à 15°C, par exemple supérieure de 5°C à 10°C, au point de fluidification de la composition, de manière à obtenir la composition fluidifiée, sous une forme bien homogène, notamment dans le cas où elle contient des cires ou des matières pulvérulentes, telles que des pigments ou des charges en assez grande quantité.

La composition fluidifiée à la température de fluidification est avantageusement introduite dans la buse de coulage en appliquant une pression allant de 100 kPa à 4000 kPa, par exemple de 500 kPa à 2500 kPa.

Pour éviter toute obstruction des moyens d'alimentation reliant la cuve et la buse, on pourra les envelopper de dispositifs à doubles parois d'eau chaude afin de maintenir la composition dans ces moyens d'alimentation à la température de fluidification souhaitée.

La composition fluidifiée peut être distribuée par des buses ou autres pièces de distribution. Une fois refroidie, elle forme un dépôt ayant une dureté appropriée à 25°C. A la température de coulage, la composition fluidifiée présente une viscosité suffisamment basse pour être distribuée à travers un orifice de la buse et permettre de déposer des feuilles, des fils ou des gouttes de faible épaisseur. La composition présente de préférence, au moment de son dépôt sur le support, une viscosité suffisamment élevée et/ou une cinétique de refroidissement telle que le dépôt garde sa forme jusqu'à sa solidification. On pourra choisir des compositions ayant une faible viscosité pour réaliser des dépôts plus précis en diminuant notamment l'épaisseur des feuilles, le diamètre moyen des fils et le diamètre moyen des gouttes.

Dans certaines applications, la composition est avantageusement dotée d'une vitesse élevée de dissipation de la chaleur, de manière à ce que le dépôt de composition se solidifie rapidement. On pourra moduler ces dissipations de chaleur en modifiant la température du support et/ou en plaçant le support dans une enceinte thermostatée.

La température de coulage de la composition dans la buse à l'étape b) peut être inférieure ou égale à la température de fluidification. Elle reste supérieure ou égale au point de fluidification de la composition.

La solidification de la surface d'un dépôt de composition s'effectue en quelques secondes lorsque le support et l'atmosphère qui l'entoure sont à 25°C. On peut décider d'accélérer la solidification du dépôt en utilisant une chambre de coulage thermostatée à une température inférieure à 25°C, par exemple égale à 10°C, 4°C, ou 0°C.

La température de coulage est par exemple comprise entre 30°C et 110°C, notamment entre 40°C et 90°C.

La température de coulage peut être inférieure ou égale à la température de fluidification. En effet, la température de fluidification permet de fondre la composition à une température où elle est suffisamment fluide pour être bien homogène. Au moment du coulage, on peut abaisser la température de la composition pour lui donner plus de consistance, pour que le dépôt formé sur le support après passage de la composition à travers la buse garde essentiellement la forme qui résulte directement de son passage à travers l'orifice de la buse. On préfère que le dépôt ne s'écoule pas sous son propre poids jusqu'à solidification complète et refroidissement à température ambiante, à une température compris entre 20°C et 25°C. Le dépôt peut néanmoins être déformé, une fois qu'il est en contact avec le support et avant solidification complète, par application ultérieure d'un autre dépôt venant à son contact.

La température de coulage sera adaptée en fonction de la vitesse de refroidissement de la composition, mais aussi en fonction de la viscosité de la composition fluidifiée pour être adaptée à un écoulement régulier à travers la buse. Un dépôt de composition se solidifie totalement de préférence en une période de temps comprise entre quelques secondes et quelques minutes.

Dans un mode de réalisation, la température de coulage est inférieure ou égale à la température de fluidification et assez proche du point de fluidification, de manière à ce que le ou les dépôts formés se refroidissent rapidement et se solidifient rapidement. En particulier les dépôts ne s'étalent pas sur le support, avant d'être solidifiés.

Par exemple, la température de coulage est supérieure d'au plus 30°C, de préférence d'au plus 20°C du point de fluidification de la composition, et inférieure d'au moins 10°C à la température de fluidification de la composition.

On peut également jouer sur la température du support pour moduler la vitesse de solidification des dépôts. Ainsi, dans un mode de réalisation, la température du support est inférieure ou égale à la température de coulage de la composition ; par exemple la température du support est de l'ordre de 20°C à 25°C. Dans un autre mode de réalisation, la température du support peut être entre 25°C à 50°C, afin d'assurer une bonne adhésion entre le support et le dépôt, ou entre les dépôts entre eux.

La viscosité dynamique de la composition, à la température de coulage et à pression atmosphérique, est de préférence comprise entre 1 mPa.s et 10 000 mPa.s, par exemple entre 100 mPa.s et 500 mPa.s.

Selon un mode de réalisation, la viscosité dynamique de la composition à la température de coulage est de préférence comprise entre 1 mPa.s et 10 000 mPa.s, par exemple entre 100 mPa.s et 500 mPa.s, ou entre 10 mPa.s et 15 mPa.s.

La viscosité de la composition peut être mesurée avec un viscosimètre Mettler RM 180 (Rheomat) peut être équipé de différents axes en fonction de l'ordre de grandeur de la viscosité que l'on souhaite mesurer. Pour une viscosité allant de 180 mPa.s à 4020 mPa.s, l'instrument est équipé d'une broche 3. Pour une viscosité allant de 1 Pa.s à 24 Pa.s, l'instrument est équipé d'une broche 4, et pour une viscosité allant de 8 Pa.s à 122 Pa.s, l'instrument est équipé d'une broche 5. La viscosité est lue sur l'instrument en unités de déviation (UA). On parle alors de tables fournis avec l'instrument de mesure, afin d'obtenir la valeur correspondante en poises. La vitesse à laquelle la broche tourne est de 200 tours par minute. A partir de ce moment, lorsque la broche est mise en rotation, à une vitesse de rotation constante imposée (en l'occurrence 200 tours par minute). La valeur de la viscosité peut varier dans le temps. Les mesures sont donc prises à des intervalles réguliers jusqu'à ce qu'elles deviennent constantes. La valeur de la viscosité devenue constante au cours du temps est la valeur retenue comme étant la valeur de la viscosité dynamique de la composition.

On pourra chercher à optimiser l'un au moins des cinq paramètres suivants du procédé de l'invention : le diamètre de buse, la distance entre la buse et le support, la vitesse des mouvements du support, la vitesse des mouvements de la buse, le débit de composition fluidifiée en sortie de la buse et la température de composition fluidifiée en sortie de la buse.

Les inventeurs ont trouvé que ces cinq paramètres permettent, pour une composition donnée, de garantir une forme régulière des dépôts, une bonne adhérence des dépôts entre eux, et une superposition des dépôts qui ne génèrent pas de déformations après le coulage.

La buse est dotée en partie inférieure d'un orifice permettant l'écoulement de la composition fluidifiée. Une des dimensions de cet orifice va de 0,01 mm à 5 mm. Elle va par exemple de 0,05 mm à 3 mm, de 0,1 mm à 1,5 mm, ou de 0,3 mm à 0,8 mm.

La forme de l'orifice peut être quelconque. Elle est de préférence circulaire. Selon un mode de réalisation, l'orifice définit un disque de 0,1 mm à 1 mm de diamètre.

L'homme du métier saura adapter la distance de la buse par rapport au support, et le débit de la composition à travers la buse, de manière à conserver l'intégrité du flux de composition à travers la buse et permettre un refroidissement rapide du dépôt lors du coulage.

Si la distance entre la buse et le support est trop faible, la géométrie des dépôts sera irrégulière. Si la distance entre la buse et le support est trop grande, le volume de composition fluidifiée sortant de la buse risque de se déformer et/ou de se solidifier avant de se déposer sur le support.

En choisissant une distance entre la buse et le support inférieure au diamètre de la buse, on peut effectuer des dépôts de composition d'épaisseurs différentes avec la même buse, dans la mesure où dans ce cas, la buse agit comme une racle. En faisant varier le débit de coulage à une hauteur de raclage donnée, on pourra également faire varier la largeur du dépôt.

Le dépôt garde généralement la forme que la composition fluidifiée avait en sortie de buse, mais il peut néanmoins être déformé, une fois qu'il est en contact avec le support et avant solidification complète, par application ultérieure d'un autre dépôt venant à son contact, notamment lorsque la distance entre la buse et le support inférieure au diamètre de la buse.

Dans le cas où l'on souhaite superposer plusieurs dépôts, on pourra chercher à maitriser leur adhérence afin de garantir la cohésion du produit cométique coulé. L'adhérence optimale entre deux dépôts pourra dépendre essentiellement de la viscosité et/ou du pourcentage de fraction liquide mentionné précédemment, de la composition à la température de coulage. L'adhérence optimale entre deux dépôts pourra également dépendre de la surface de la zone de contact entre deux dépôts. Cette surface pourra être modulée en fonction de la distance entre la buse et le support, et/ou de la température de coulage.

On pourra chercher à adapter la vitesse de déplacement relative entre la buse et le support. En effet, si elle est trop élevée, les dépôts risquent d'être discontinus et/ou d'avoir un diamètre inférieur à celui de la buse, ce que l'on préfère éviter car cela introduirait des défauts dans le produit coulé.

La vitesse de déplacement relatif de la buse par rapport au support pendant le coulage peut aller de 0 mm/s à 300 mm/s, par exemple de 100 mm/s à 150 mm/s.

Pour des dépôts de très petit volume, comme les gouttes, la buse est de préférence immobile par rapport au support au moment de l'éjection de la goutte par la buse.

Pour les dépôts en forme de fil, on choisira la vitesse de déplacement de la buse et le débit de coulage en fonction de l'effet recherché mais aussi de la viscosité de la composition à la température de coulage.

On adapte avantageusement le débit et la température de coulage en fonction du diamètre de buse choisi, de manière à ce que le dépôt se solidifie très rapidement après coulage de la composition.

La composition fluidifiée est coulée à un débit contrôlé, à partir d'une buse de coulage, sur le support, de préférence selon un motif prédéterminé. Les dépôts - qui ont été effectués par la répétition de cycles de coulage comprenant les étapes a) à e) - se solidifient et adhèrent avantageusement les uns aux autres pour constituer un produit cosmétique en relief et/ou comportant une pluralité de teintes.

Le débit est par exemple compris entre 0,05 mm³/s et 2 mm³/s pour une buse de 0,1-1 mm de diamètre. Le débit de coulage peut être compris entre 0,1 mm³/s et 1 mm³/s.

Le débit est contrôlé de sorte qu'un premier dépôt d'au moins une première composition soit partiellement solidifié avant qu'un deuxième dépôt soit coulé au contact du premier.

Le débit peut être également adapté en fonction de la vitesse relative de déplacement de la buse par rapport au support.

La température de la composition fluidifiée en sortie de la buse, encore appelée température de coulage pourra être choisie de manière à obtenir une viscosité relativement faible pour une bonne extrusion de la matière et une viscosité suffisamment élevée pour obtenir un dépôt de forme régulière pendant tout la durée du coulage en sortie de buse.

Le procédé peut comprendre le coulage de plusieurs dépôts à travers plusieurs buses de façon concomitante. Les coulages simultanés sont dans ce cas de préférence réalisés à des coordonnées du support différentes. Les buses peuvent être solidaires les unes des autres et se déplacer selon des trajectoires parallèles, ou au contraire être dotée chacune de moyens de déplacement propres de manière à suivre des trajectoires indépendantes les unes des autres.

Le procédé peut également comprendre le coulage d'un dépôt de composition et le coulage d'un dépôt de matériau thermoplastique de façon concomitante ou séparée.

Le procédé de l'invention peut comprendre une étape g) de coulage d'une matière thermoplastique. Cette étape peut être réalisée antérieurement, postérieurement ou de façon simultanée à l'étape c) de coulage de la composition cosmétique.

La matière thermoplastique peut être par exemple choisie parmi les suivantes : polyéthylène (PE), polychlorure de vinyle (PVC), polypropylène (PP), polystyrène (PS), polyméthacrylate de méthyle (PMMA), polyéthylène téréphtalate (PET), acide polylactique (PLA), polyamide (PA), acrylonitrile butadiène styrène (ABS), ainsi que les copolymères des précédents.

On peut couler un matériau thermoplastique en même temps que la composition cosmétique, mais en utilisant une autre buse. Dans ce mode de réalisation, on choisira de préférence des dépôts en forme de gouttes de 0,4 à 0,7 mm, permettant notamment de réaliser des dégradés très fins.

Le support définit avantageusement une surface sur laquelle le ou les dépôts de composition sont coulés. La surface est essentiellement plane en ce sens que son rayon de courbure est faible (inférieur à 20°). Cette surface ne définit pas un volume que le dépôt de composition vient remplir. Le support ne remplit pas la fonction de moule, contrairement aux supports utilisés dans l'art antérieur, notamment pour la fabrication de rouges à lèvres en bâton et en coupelle.

Le support peut être de nature cosmétique ou non cosmétique.

La température du support peut être inférieure ou égale à 25°C, par exemple égale à 10°C, 4°C, ou 0°C, dans le cas où l'on souhaite accélérer le refroidissement du dépôt de composition coulée. Dans l'objectif inverse, on peut chauffer le support à une température supérieure à 25°C.

Selon un mode de mise en œuvre préféré, le support est de nature cosmétique et peut être appliqué sur la peau au même titre que la composition qui a été coulée dessus. Le support peut être un rouge à lèvres, un produit parfumant, un fond de teint solide ou un fard à paupière

Par exemple, le support est un produit cosmétique qui a été fabriqué par moulage ou pressage préalablement à la mise en œuvre du procédé de l'invention. Dans ce mode de réalisation, le support peut être réalisé par fusion d'un mélange comprenant majoritairement une phase grasse solide à 25°C, par coulage dudit mélange dans un moule, par refroidissement dudit mélange, puis par démoulage de la pièce ainsi formée.

L'invention présente l'avantage de permettre l'assemblage de plusieurs produits cosmétiques qui sont mis en forme de façon indépendante.

Le produit obtenu par le procédé de l'invention peut comprendre l'assemblage d'un support cosmétique anhydre et de dépôts anhydres, d'un support anhydre et de dépôts en émulsion eau-dans-huile, d'un support en émulsion eau-dans-huile et de dépôts anhydres, d'un support en émulsion eau-dans-huile et de dépôts en émulsion eau-dans-huile.

Le support de nature non cosmétique peut être une pièce en matière métallique ou en matière plastique. Il peut également constituer un récipient servant de conditionnement au produit cosmétique.

Le support peut rester solidaire du produit cosmétique obtenu à l'issue de l'étape f), ou au contraire en être séparé. Le support est ainsi destiné à être séparé du produit cosmétique, ou constitue au contraire une partie du produit cosmétique.

L'étape f) peut être effectuée plusieurs fois. En effet, les étapes a) à e) peuvent être répétées une ou plusieurs fois de manière à former plusieurs dépôts sur le support. Ces dépôts peuvent être en contact les uns des autres, ou au contraire déposé de manière disjointe sur le support. Pour cela, la buse est déplacée dans un plan parallèle au plan moyen du support ou dans un plan perpendiculaire à celui-ci.

Selon le procédé de l'invention, la composition et le diamètre de la buse sont choisis de telle sorte qu'il n'est pas nécessaire d'attendre qu'un premier dépôt soit partiellement ou totalement solidifié, avant d'effectuer un deuxième dépôt. Au contraire, on préfère que le premier dépôt ne soit pas solidifié lorsqu'un deuxième dépôt est coulé et entre en contact avec ce dernier, de manière à promouvoir leur adhésion.

La composition utilisée dans le cadre du procédé de coulage de l'invention est solide à 25°C et contient au moins une huile. L'huile ou le mélange d'huiles est de préférence le composé majoritaire de la composition.

Cette composition permet d'effectuer des dépôts, en particulier des dépôts présentant une très faible épaisseur, parce qu'elle est dotée d'une fluidité suffisante et d'une consistance suffisamment élevée pour que le dépôt ne soit pas déformé après passage à travers l'orifice de la buse, pour que le dépôt ne s'affaisse pas une fois coulé sur le support avant d'être solidifié, et pour que le dépôt adhère au support ou à un dépôt préalablement réalisé.

Par exemple, la dureté de la composition - mesurée selon l'une des méthodes décrites ci-après est comprise entre 2 N et 70 N, de préférence encore compris entre 3 N et 45 N. La dureté de la composition peut être comprise par exemple entre 18 N et 44 N, ou entre 30 N et 40 N. Dans certains modes de réalisation, la dureté de la composition est entre 15 N et 25 N, entre 6 N et 17 N ou entre 9 N et 15 N.

La dureté des compositions peut être mesurée avec un texturomètre de la marque Stable Micro Systems, modèle TA.XT.Plus, par pénétration d'une sonde hémisphérique de diamètre 12.7 mm. La mesure a été réalisée dans un pot Cléopâtre que l'on a rempli de 100 ml de composition. On prend de préférence la moyenne de trois mesures effectuée sur trois échantillons différents en choisissant les paramètres de mesure suivants : approche à 2 mm/s, mesure à 2 mm/s, retrait à 2 mm/s, pénétration de 13 mm, relaxation de 25 sec, et force déclenchante de 0,02 N.

La dureté de la composition peut aussi être mesurée avec un texturomètre TA- XT2i de chez Rhéo, équipé d'un cylindre en ébonite de 25 mm de hauteur et de diamètre 8 mm. La mesure de dureté peut être effectuée au centre de 5 échantillons de composition. Le cylindre est introduit dans chaque échantillon de composition à une pré- vitesse de 2 mm/s puis à une vitesse de 0,5 mm/s et enfin à une vitesse de retrait de 2 mm/s. Le déplacement total est de 1 mm.

La composition peut répondre aux paramètres suivants :
- une dureté comprise entre 15 N et 25 N et un point de goutte compris entre 70°C et 80°C, ou
- une dureté comprise entre 6 N et 17 N et un point de goutte compris entre 75°C et 85°C, ou
- une dureté comprise entre 9 N et 15 N et un point de goutte compris entre 55°C et 60°C, ou
- une dureté comprise entre 18 N et 44 N, un point de goutte compris entre 40°C et 70°C, une température T50 comprise entre 70°C et 85°C, et une température T90 comprise entre 74°C et 81°C, ou
- une dureté comprise entre 30 N et 40 N, un point de goutte compris entre 55°C et 60°C, une température T50 comprise entre 75°C et 80°C, et une température T90 comprise entre 75°C et 80°C.
- une dureté entre 40 N et 45 N et un point de goutte compris entre 75°C et 80°C.

Le procédé de l'invention peut comporter des traitements qui sont effectués au cours de l'une des étapes a) à f), ou dans une étape séparée, afin d'améliorer la qualité esthétique du produit. On peut par exemple réaliser un flammage infrarouge des dépôts réalisés successivement par le procédé pour lisser les surfaces créées par leur juxtaposition, leur superposition ou leur chevauchement.

D'autres traitements de surface connus dans l'état de l'art, tels que la création de motifs par laser, la pulvérisation de nacres ou encore l'électrodéposition de nacres, peuvent être envisagées.

Le produit cosmétique obtenu par fusion d'une composition solide à 25°C contenant une huile, et par au moins un dépôt de ladite composition à l'état fluide, a la forme d'une feuille, d'un fil ou d'une goutte. On peut prévoir que le produit comporte plusieurs dépôts de formes différentes.

Le produit cosmétique multi-teinte, multi-texture ou multi-matière, est notamment un produit de maquillage, un produit de soin, un produit de protection solaire ou un produit parfumant, comprenant au moins un dépôt sur un support d'une composition cosmétique solide contenant au moins une huile qui est de préférence dotée d'un point de fluidification compris entre 30°C et 150°C, et qui a une dureté moyenne - mesurée à 25°C à l'aide d'un texturomètre muni d'une sonde hémisphérique - comprise entre 0,5 Net 80 N, le dépôt ayant la forme de fils et/ou de gouttes d'épaisseur inférieure à 5 mm.

Le produit comprend de préférence au moins deux dépôts d'une composition cosmétique. Il peut également comprendre plusieurs dépôts de compositions cosmétiques différentes.

Le procédé de l'invention permet de fabriquer un produit cosmétique multi-teinte, multi-texture ou multi-matière, notamment un produit de maquillage, un produit de soin, un produit de protection solaire ou un produit parfumant, comprenant une pluralité de domaines constitués de compositions cosmétiques différentes solides à 25°C contenant chacune indépendamment l'une de l'autre au moins une huile, les compositions étant dotées indépendamment l'une de l'autre d'un point de fluidification compris entre 30 °C et 150°C, et d'une dureté moyenne - mesurée à 25°C à l'aide d'un texturomètre muni d'une sonde hémisphérique - comprise entre 0,5 N et 80 N, le produit ne comprenant ni un empilement de couches planes parallèles entre elles, ni une succession de couches cylindriques coaxiales, ni une surface cosmétique plane. Par surface cosmétique plane, on entend au moins une surface libre d'une composition cosmétique du produit obtenu par le procédé de l'invention qu'un utilisateur peut prélever.

Le produit cosmétique obtenu par le procédé de l'invention peut comprendre une pluralité de domaines de compositions en contact les uns avec les autres qui ont des points de goutte et des duretés différentes. Ces domaines peuvent décrire des motifs géométriques ou des motifs non géométriques. Leurs teintes peuvent être identiques ou différentes.

Le produit peut prendre des formes très variées, telles qu'un stick, une demi-sphère ou une sphère.

La surface ou la masse du peuvent par exemple présenter des formes structurées multicolores non géométriques. Par « forme structurée », on entend un motif en deux dimensions dont la forme n'est pas aléatoire.

Le produit peut comprendre des formes ajourées, des entrelacements de rubans comme des tresses, des surfaces poreuses, ou des dégradés de couleurs.

Le produit cosmétique peut avoir une ou plusieurs fonctionnalités, par exemple être un produit de maquillage, un produit de soin, un produit de protection solaire ou un produit parfumant. Parmi les produits de maquillage, on peut citer les produits de maquillage des lèvres, des yeux, des joues, des sourcils, du visage, et du corps, par exemple un rouge à lèvres, un fard à joues ou un fard à paupières. Dans un mode de réalisation, le produit cosmétique peut être utilisé comme fard à paupières, fard à joues, brillant à lèvres, baume à lèvres, rouge à lèvres, fond de teint. Il peut également être doté de plusieurs de ces fonctionnalités.

Le produit cosmétique obtenu par le procédé de l'invention peut comprendre une composition cosmétique dont les reliefs ont une hauteur inférieure à 1 mm, de préférence inférieure à 0,5 mm, de préférence encore inférieure ou égale à 0,1 mm, ou telle que sa surface de présentation à l'utilisateur présente des zones de courbure très élevée. Ces motifs en relief ne pouvaient pas être obtenus auparavant par un procédé de coulage classique dans un moule.

Le produit cosmétique peut comprendre plusieurs compositions décrites précédemment. Dans un mode de réalisation particulier, le produit comprend l'assemblage i) d'un raisin de rouge à lèvres, et ii) d'au moins un dépôt de la même composition que le raisin de rouge à lèvres à l'exception des nacres et des pigments qu'elle contient.

Le support du produit cosmétique peut être une poudre pressée réalisée une des séries d'étapes suivantes : i) compactage à l'aide d'une empreinte d'un mélange comprenant une proportion majoritaire de poudres, afin de former des cakes de poudre, ii) compactage d'un mélange comprenant une proportion majoritaire de poudres, et découpe laser selon la forme souhaitée, ou iii) mélange de poudres dans un solvant afin d'obtenir une pâte qui est extrudée, découpage de la pâte à l'emporte-pièce selon la forme souhaitée, et séchage.

Le produit cosmétique peut comprendre un conditionnement tel qu'un flacon ou un boîtier. Le boîtier peut comprendre un couvercle qui peut être articulé sur un fond, et comprendre un miroir. Le fond peut comporter un évidement dans lequel est placé un applicateur, sous forme d'un pinceau, d'une brosse ou d'un embout en mousse fixé à une tige. L'applicateur peut être logé et encliqueté dans le boîtier.

Le conditionnement peut être doté de un ou de plusieurs compartiments ou évidements. Un de ces compartiments peut contenir des compositions différentes qui sont les unes en contact des autres, sans qu'il soit nécessaire de les séparer par des parois. Au contraire, dans l'art antérieur, chaque compartiment est destiné à recevoir une seule composition à la fois, compte-tenu des contraintes de fabrication des procédés de coulage.

La composition cosmétique peut être anhydre, ou contenir à la fois une phase aqueuse et une phase grasse. La composition peut être par exemple une émulsion eau-dans-huile solide à température ambiante. Ladite composition contient de préférence majoritairement des corps gras, tels que des huiles, des cires et des composés gras pâteux.

La composition utilisée dans le procédé de l'invention contient une huile, qui peut être volatile ou non.

Les huiles volatiles peuvent être choisies parmi l'isododécane, l'isodécane, l'isohexadécane, l'octaméthyl cyclotétrasiloxane, le décaméthyl cyclopentasiloxane, le dodécaméthyl cyclohexasiloxane, le pentacyclométhicone.

Comme huile hydrocarbonée non volatile, on peut notamment citer les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique ; les éthers de synthèse ayant de 10 à 40 atomes de carbone; la vaseline, les polydécènes, le polyisobutène hydrogéné, les polybutylènes, les polyisobutylènes hydrogénés, les polydécènes hydrogénés, les copolymères de la vinylpyrrolidone tel que le PVP/ héxadécène copolymère, le tétrapélargonate de pentaérythrityle, le triisostéarate de polyglycérol-2, le tridécyl trimellitate, le citrate de triisoarachidyle, le tétraisononanoate de pentaérythrityle, le triisostéarate de glycéryle, le tétraisostéarate de pentaérythrityle et le tridécyl-2 tétradécanoate de glycéryle, le squalane, les esters de synthèse comme par exemple l'octanoate de cétostéaryle, le myristate d'isopropyle, le palmitate d'isopropyle, les benzoates d'alcools en C12 à C15, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; esters de polyols et les esters du pentaérythritol, - les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol, - les acides gras supérieurs tels que l'acide oléique, l'acide linoléique, l'acide linolénique et leurs mélanges.

Les huiles de silicone non volatiles utilisables dans la composition peuvent être les polydiméthylsiloxanes (PDMS) non volatiles ou les phényl triméthicones.

Les huiles peuvent représenter de 0,01 à 99 % du poids total de la composition, de préférence de 0,05 à 60 % et mieux de 1 à 35 %.

La composition peut contenir un composé structurant qui donne à l'huile qu'elle contient une consistance solide à 25°C. Ce composé structurant peut être choisi parmi les cires, les corps gras pâteux, les polymères gélifiants d'huile et les composés minéraux gélifiant d'huile.

La composition peut comprendre un mélange d'huile(s), de cire(s), de pâteux, de pigment(s) et de charge(s). Par exemple, les huiles représentent entre 55 et 65% en poids de la composition, tandis que les cires représentent de 2 à 20% en poids de la composition et les pâteux représentent de 10 à 30% en poids de la composition. Une autre composition sera telle que les huiles représentent entre 55 et 65% en poids de la composition, tandis que les cires représentent de 5 à 11% en poids de la composition et les pâteux représentent de 20 à 30% en poids de la composition.

Le terme "cire", au sens de la présente invention, entend désigner un composé solide à 25 °C qui présente un changement d'état solide/liquide réversible et une température de fusion supérieure à 30 °C, de préférence supérieure à 45°C. On peut citer les cires microcristallines, les cires de paraffine, les cires de polyéthylène, l'ozokérite, la cire de carnauba, la cire d'abeilles, les produits comprenant un mélange de polyéthylène et d'alcools comportant 20 à 50 atomes de carbone, les cires de silicone notamment les alkyl diméthicones, les stéarates d'alkyle en C20-C40, les cires obtenues par hydrogénation catalytique d'huiles végétales ayant des chaînes linéaires ou ramifiées en C8-C32 telles que l'huile de jojoba hydrogénée, les cires obtenues par hydrogénation de l'huile de ricin estérifiée avec un alcool gras, la cire de candelilla, les copolymères d'anhydride maléique et d'alpha-oléfine, les cires obtenues par catalyse métallocène et la cire de lanoline.

Le terme "composé gras pâteux" désigne un composé gras non cristallin comprenant à une température de 25°C, une fraction liquide et une fraction solide. Le composé pâteux est par exemple choisi dans le groupe constitué de la lanoline et de ses dérivés, les composés siliconés polymères, les copolymères de (méth)acrylates d'alkyles ayant de préférence un groupement alkyle en C8-C30, les oligomères homo et copolymères d'esters vinyliques ayant des groupements alkyles en C8-C30, les oligomères homo et copolymères de vinyl-éthers ayant des groupements alkyles en C8-C30, les polyéthers liposolubles résultant de la polyéthérification entre un ou plusieurs diols en C2-C50, les copolymères d'éthylène-oxyde et/ou de propylène-oxyde avec des alkylènes-oxydes à longue chaîne en C6-C30, les esters de diglycérol, le propionate d'arachidyle, les esters de phytostérol, les polyesters non réticulés résultant de la polycondensation entre un diacide ou un polyacide carboxylique linéaire ou ramifié en C4-C50 et un diol ou un polyol, l'ester résultant de la réaction d'estérification de l'huile de ricin hydrogénée avec l'acide isostéarique tel qu'un mono- di- ou tri-isostéarate d'huile de ricin hydrogénée, un mélange de stérols de soja et de pentaérythritol oxyéthyléné (5 OE) oxypropyléné (5 OP), et leurs mélanges.

Un agent gélifiant l'huile est par exemple choisi parmi des polyamides, tels que des copolymères polyamides à terminaison ester (Uniclear®, Union Carbide), ou bien des polyamides siliconés, ou bien encore des dérivés de la L-Glutamide tels que le dibutyl lauroyl glutamide, commercialisé par Ajinomoto ou un de leurs mélanges, des silices pyrogénées, des argiles, des copolymères Diisostearyl Malate (and) Bis-Dioctadecylamide Dimer Dilinoleic Acid/Ethylenediamine Copolymer tel que l'Haimalate PAM® commercialisé par Kokyu Alcohol Kogyo ou bien encore des copolymères comprenant au moins une unité styrène, hydrogénés ou non hydrogénés, tels que par exemple un copolymère hydrogéné styrène/méthylstyrène/indène, le palmitate de dextrine, les copolymères blocs (diblocs, triblocs, étoile) styrène / butylène / styrène ou styrène / éthylène propylène / styrène. La composition pourra par exemple contenir de 10 à 15% en poids de cire(s), de 0 à 10% en poids de pâteux, et de 60 à 70% en poids d'huile(s).

La composition comprend par exemple un gel anhydre contenant une huile et un agent gélifiant de cette huile tel que décrit dans le document FR 2 958 159 ou FR 2 975 589. Selon un mode de réalisation, l'agent gélifiant est un polymère de type polyamide à terminaison amide, de préférence un polymère de type polyamide à terminaison amide tertiaire (ATPA).

On peut utiliser comme huile le mélange d'une huile non volatile de polyalkylène hydrogéné, notamment un polyisobutène hydrogéné et d'un ester d'acide gras comprenant au moins un groupe hydroxyl libre, par exemple un ester d'hydroxystéarate, de préférence l'hydroxystéarate d'éthylhexyle, lorsque l'agent gélifiant est un ATPA.

Les textures de type émulsion solide sont de préférence des émulsions dont la phase aqueuse peut comprendre des solvants organiques miscibles à l'eau comme l'éthanol, l'isopropanol, le propylène glycol, l'éthylène glycol, le 1,3-butylène glycol, le dipropylène glycol, les cétones en C3-C4, et les aldéhydes en C2-C4.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids par rapport au poids total de la composition. Cette phase aqueuse peut, le cas échéant, être épaissie, gélifiée ou structurée en y incorporant en outre un gélifiant aqueux connu de l'homme du métier.

La composition peut contenir des pigments minéraux, organiques ou nacrés, et éventuellement des charges.

Parmi les pigments minéraux, on peut citer, à titre d'exemples le dioxyde de titane, éventuellement traité en surface ; les oxydes de fer noir, jaune, rouge et brun ; le violet de manganèse ; le bleu outremer ; l'oxyde de chrome ; l'oxyde de chrome hydraté et le bleu ferrique.

Parmi les pigments organiques, on peut citer, par exemple, les pigments D & C red n° 19 ; D & C red n° 9 ; D & C red n° 21 ; D & C orange n° 4 ; D & C orange n° 5 ; D & C red n° 27 ; D & C red n° 13 ; D & C red n° 7 ; D & C red n° 6 ; D &C yellow n° 5 ; D & C red n° 36 ; D & C orange n° 10 ; D & C yellow n° 6 ; D & C red n° 30 ; D &C red n° 3 ; le noir de carbone et les laques à base de carmin de cochenille.

Les pigments nacrés peuvent être choisis notamment parmi les pigments nacrés blancs, tels que le mica recouvert d'oxyde de titane, l'oxychlorure de bismuth; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Les charges peuvent être choisies parmi le talc, un silicate de magnésium hydraté; les micas de dimensions de 2 à 200 µm; le kaolin, un silicate d'aluminium hydraté; les oxydes de zinc et de titane ; le carbonate de calcium, le carbonate et l'hydrocarbonate de magnésium ; la silice ; le stéarate de zinc, de magnésium ou de lithium, le laurate de zinc, le myristate de magnésium ; les poudres de polymères synthétiques, tels que le polyéthylène, les polyesters et les polyamides (par exemple le nylon); la silice sphérique ; les dioxydes de titane sphériques; les billes de verre et de céramique ; des poudres de matériaux organiques d'origine naturelle comme les amidons de maïs, de blé, de riz, réticulées ou non; des poudres de polymères de synthèse, réticulées ou non, sphéronisées, comme des poudres de polyamide telles que des poudres de poly-β-alanine et des poudres de nylon, des poudres d'acides polyacrylique ou polyméthacrylique, des poudres de polystyrène réticulées par le divinylbenzène, des poudres de résine de silicone, des poudres de téflon.

La composition peut contenir un actif cosmétique choisi parmi les vitamines A, E, C, B3, les provitamines comme le D-panthénol, le phosphate de tocophérol, les actifs apaisants comme l'alpha-bisabolol, l'aloe vera, l'allantoïne, les agents restructurants, le menthol, le miel, les émollients, les agents hydratants, les actifs antirides, les actifs repulpants et tenseurs, et leurs mélanges.

La composition peut comprendre d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les filtres solaires, les tensioactifs, les émulsionnants, les agents dispersants, les antimousses, les neutralisants, les stabilisants et leurs mélanges.

Des produits cosmétiques, en particulier des produits cosmétiques de maquillage de la peau, peuvent être obtenus selon le procédé décrit précédemment.

Le dépôt ou la séquence de dépôts de composition sont avantageusement réalisées grâce à des moyens de commande numérique. Dans ce cas, la ou les buses de coulage sont reliées sont commandées par un logiciel permettant de programmer les coordonnées spatiales et temporelles des dépôts.

Le coulage de la composition sur le support peut se faire selon un modèle pré-existant qui se trouve sous la forme d'un fichier numérique. Dans un mode de réalisation de l'invention, on réalise préalablement au coulage de la composition, le dessin en deux ou en trois dimensions du produit cosmétique que l'on souhaite fabriquer. Ce dessin prend avantageusement la forme d'un fichier numérique que l'on peut convertir en coordonnées de dépôts successifs.

Dans un mode de réalisation, un objet est créé à l'aide d'un logiciel de modélisation sous la forme d'un fichier numérique. Ce fichier peut représenter un maillage, et être sous un format .STL ou .OBJ. Cependant d'autres formats peuvent contenir davantage d'informations sur l'objet, comme les couleurs et les matériaux qui le composent. Le fichier numérique peut être constitué d'une enveloppe polygonale (ou maillage) représentant l'objet virtuel numérique.

Le fichier numérique peut être ensuite ouvert dans un logiciel éditeur de G-code. Le G-code est un langage de programmation numérique qui permet de piloter les moteurs de l'appareil. Le fichier numérique de représentation du modèle à reproduire peut être converti en G-code à l'aide de logiciels connus de l'homme du métier. Le G-code contient toutes les informations relatives aux coordonnées et la séquence des déplacements du support et de la buse de coulage.

Ainsi, dans un mode de réalisation particulier de l'invention, le procédé met en œuvre un support motorisé, une buse de coulage motorisée, un moyen d'alimentation en composition cosmétique fluidifiée, des moteurs pas à pas, une carte de contrôle, un logiciel permettant de convertir un objet numérique en G-Code, et un logiciel adapté pour transmettre le G-code à la carte de contrôle.

Le procédé de l'invention permet ainsi de réaliser de manière reproductible des formes identiques et conformes à un modèle en deux ou trois dimensions.

La buse de coulage est de préférence associée à un moyen d'alimentation en composition. Ce moyen peut être une seringue munie d'un piston motorisé, une vis ou un différentiel de pression. Ce moyen d'alimentation peut également permettre de régler le débit de coulage de la composition.

Dans un mode de réalisation, le moyen d'alimentation choisi est une seringue ; le piston de la seringue est couplé à un système vis-écrou-engrenages alimenté par un moteur pas à pas. Lorsque le moteur tourne dans un sens, le piston pousse la matière hors du nez de la seringue et lorsqu'il tourne dans le sens contraire, le piston remonte et retient la matière pour l'empêcher de couler par gravité lorsque le coulage est arrêté.

L'appareil ou la buse sont avantageusement dotés de moyens pour contrôler le débit de la composition fluidifiée sortant de la buse.

L'appareil peut donc comprendre un dispositif de commande servant à actionner des moteurs permettant la mise en mouvement de la buse et/ou du support dans au moins deux des trois directions de l'espace.

L'appareil utilisé pour la mise en œuvre du procédé de l'invention comprend au moins une buse de coulage dotée d'une ouverture ou orifice, laquelle buse est reliée à des moyens mécaniques et des moteurs permettant de la déplacer dans les trois directions de l'espace.

Les moyens mécaniques sont entrainés par les moteurs pour déplacer la buse et le support, selon un trajet avantageusement prédéterminé, au cours de l'étape de coulage c) ou après l'arrêt du coulage à l'étape d) pour déplacer la buse à un autre endroit du support et effectuer un nouveau dépôt, ou utiliser une autre buse pour couler une autre composition au même endroit.

Selon un mode de réalisation, la buse est entraînée dans un mouvement vertical (axe z), et le support est déplacé sur des rails selon un trajet horizontal (axe y et axe x).

Les mouvements peuvent être effectués dans les trois directions de l'espace au moment du coulage, mais aussi une fois le débit annulé, afin d'obtenir la forme ou l'arrangement spatial de matières et/ou de teintes voulus.

Ces mouvements mécaniques sont de préférence obtenus par des signaux de commande envoyés par un système CAD/CAM (ordinateur/contrôleur). Dans un tel système, la conception du produit cosmétique est effectuée sur un ordinateur, puis un logiciel convertit la forme en trois dimensions en une séquence de données. Ces données sont transmises aux moteurs d'entraînement sous forme de signaux de commande par l'intermédiaire d'une machine de commande assistée par ordinateur.

Plusieurs moyens de chauffage servent à maintenir la composition décrite précédemment à l'état fluide, à la température de fluidification et à la température de coulage.

L'appareil peut éventuellement comprendre au moins un moyen de contrôle de la température du support, un moyen de contrôle de la température de la composition, et/ou un moyen de contrôle de la température de l'atmosphère, de manière à moduler la vitesse de solidification du dépôt de composition. La modulation de cette vitesse peut être l'accélération ou le ralentissement et permettre par exemple à un premier dépôt d'adhérer à un deuxième dépôt qui serait effectué postérieurement, avant que le premier dépôt soit totalement solidifié.

Au cours de l'étape a) du procédé de l'invention, la composition est fluidifiée par chauffage à une température de fluidification supérieure ou égale à son point de fluidification. L'appareil utilisé dans le cadre du procédé comprend donc un dispositif de chauffage en amont de la buse de coulage pour liquéfier la composition. Ce moyen de chauffage est avantageusement associé à une cuve maintenant la composition fluidifiée sous agitation à une température constante.

Au cours de l'étape b) du procédé de l'invention, la composition est de préférence maintenue à une température supérieure à son point de fluidification par chauffage. L'appareil utilisé dans le cadre du procédé comprend donc de préférence un dispositif de chauffage et de contrôle en température situé au niveau de la buse de coulage.

Le procédé de l'invention peut être mis en œuvre sur un site de fabrication de produits cosmétiques, mais aussi sur un point de vente ou dans un salon de beauté.

Lorsque le procédé est mis en œuvre sur un lieu public, le consommateur peut être consulté préalablement à l'étape a) afin de choisir les fonctionnalités, la forme et/ou la teinte du produit cosmétique qu'il souhaite. Dans ce cas, il peut fournir une photo ou un dessin qui sera reproduit sur ordinateur avant d'être converti en données de commandes pour réaliser les dépôts successifs.

### BREVE DESCRIPTION DES DESSINS

La Fig. 1 est une vue schématique d'un produit cosmétique multi-teinte obtenu par le procédé de l'invention.
La Fig. 2 est une vue schématique d'un produit cosmétique multi-texture obtenu par le procédé de l'invention.

L'invention est illustrée par les exemples suivants. Sauf indication contraire, les valeurs indiquées dans le cadre de la présente invention résultent de mesures réalisées à 25°C et à 1 atm (10⁵ Pa).

### EXEMPLE 1: Rouae à lèvres multi-teinte

### Préparation des compositions

On prépare trois compositions de teintes différentes : une rouge, une violette et une rose. Les trois compositions diffèrent par la nature et la proportion relative des pigments et nacres. Les ingrédients qui les composent, ainsi que leurs proportions exprimées en pourcentages massiques, sont reproduits ci-après.

| | |
|---|---|
| Mélange de polyéthylène, de cire d'abeille et d'ozokérite | 12,7 |
| Mélange de Bentone et de C₁₀-C₃₀ esters | 3,4 |
| Mélange de polyisobutène, esters, polydécène et octyldodécanol | 64,5 |
| Mélange (1) de Pigments et nacres | |
| ou Mélange (2) de Pigments et nacres | |
| ou Mélange (3) de Pigments et nacres | 15,9 |
| Conservateurs, agents actifs ou parfums | qsp 100 |

Les compositions sont obtenues par mélange des ingrédients sous agitation à une température égale à 95°C. Les compositions sont refroidies à 25°C. On mesure ensuite la dureté et le point de goutte des compositions par les méthodes suivantes.

La dureté des compositions a été mesurée avec un texturomètre de la marque STABLE MICRO SYSTEMS, modèle TA.XT.Plus, par pénétration d'une sonde hémisphérique de diamètre 12.7 mm (appelée doigt Delrin). La mesure a été réalisée dans un pot Cléopâtre de 100 ml, et elle a été répétée trois fois.

Les paramètres de la mesure étaient les suivants :
- Approche 2 mm/s
- Test 2 mm/s
- Retrait 2 mm/s
- Pénétration 13 mm
- Relaxation 25 sec
- Force déclenchante : 2 g.

Le point de goutte a été mesuré avec un appareil Mettler Toledo DP70 doté de cupules normalisées diamètre d'orifice 2,8 mm, en adoptant une vitesse de chauffe de 2,0 °C/min, une température de départ de 40 °C, et une température de fin de 95 °C. La goutte est détectée visuellement à l'aide d'une caméra intégrée à l'appareil.

La première composition a une dureté de 43,3 N et un point de goutte égal à 78,2°C. La deuxième composition a une dureté de 40,2 N et un point de goutte égal à 79,4°C. La troisième composition a une dureté de 40,7 N et un point de goutte égal à 77,5°C.

### Appareil de coulage

L'appareil de coulage comporte un support plan non cosmétique, trois buses et un moyen d'alimentation pour chaque buse. Chaque buse présente un orifice circulaire de diamètre 0,5 mm ; elle est reliée à des moyens mécaniques permettant son déplacement selon une trajectoire verticale. Le support est muni de moyens mécaniques permettant son mouvement dans le plan qu'il définit.

L'appareil de coulage est placé dans une enceinte thermostatée à 25°C ; il est relié à un ordinateur qui commande les moyens d'alimentation des buses, le déplacement des buses et le déplacement du support, pour fabriquer un stick de rouge à lèvres à trois teintes présentant des motifs géométriques, qui ont été prédéfinis.

### Procédé de coulage

Dans ce procédé, les compositions sont coulées par dépôts successifs selon une séquence prédéterminée programmée par un logiciel de commande.

La première composition est fluidifiée à une température de fluidification de l'ordre de 90-95°C, introduite dans la première buse par un premier moyen d'alimentation, puis coulée à pression atmosphérique sur le support pour former un premier fil de la première composition. La forme du fil déposé est définie par le mouvement du support, qui peut suivre une trajectoire linéaire ou courbe pendant le coulage. Le coulage du premier dépôt de la première composition est arrêté par commande informatique du moyen d'alimentation de la première buse.

On vient ensuite positionner la deuxième buse et le support de manière à effectuer un premier fil de la deuxième composition, dans les mêmes conditions que celles qui ont été décrites précédemment pour effectuer le premier dépôt de la première composition. Le coulage du premier dépôt de la deuxième composition est ensuite arrêté.

Le programme de commande des moyens d'alimentation des trois buses définit la séquence et les durées de coulage des trois compositions en fonction des motifs et des formes que l'on souhaite obtenir.

Par exemple, on pourra réaliser la série d'étapes suivantes :
Etape i) : couler le premier dépôt de la première composition, puis le premier dépôt de la deuxième composition sur le support, à une distance constante entre le support et les buses, pour ensuite
Etape ii) : augmenter la distance entre le support et les buses d'une longueur égale à l'épaisseur des premiers dépôts, et couler le premier dépôt de la troisième composition, et le deuxième dépôt de la première composition sur les dépôts réalisés à l'étape précédente, puis
Etape iii) : augmenter la distance entre le support et les buses d'une longueur égale à l'épaisseur des dépôts réalisés à l'étape ii), et couler le deuxième dépôt de la deuxième composition, et le deuxième dépôt de la troisième composition sur les dépôts réalisés à l'étape ii),
Etape iv) : répéter les étapes i) à iii) en augmentant la distance entre le support et les buses entre chaque étape, de manière à garder constant, d'une étape à l'autre, l'écart entre la buse et la surface sur laquelle les dépôts doivent être effectués.

Une fois la séquence d'étapes terminée, le produit constitué des dépôts successifs est séparé du support de l'appareil.

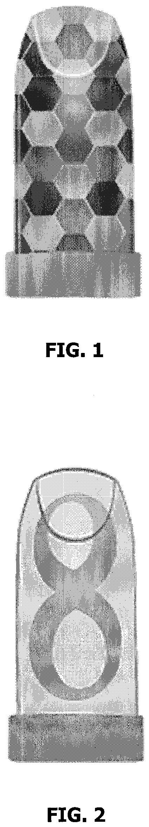

En suivant cette séquence, le produit obtenu peut être un rouge à lèvres en forme de stick tel que représenté sur la Figure 1. Ce rouge à lèvres est constitué de domaines en forme de dodécaèdre. Le choix des teintes est tel que l'ensemble restitue un ensemble harmonieux et esthétique.

### EXEMPLE 2: Baume à lèvres multi-texture

### Préparation des compositions

On prépare une composition (1) transparente selon le même procédé que celui décrit à l'Exemple 1. Les ingrédients qui la composent, ainsi que leurs proportions exprimées en pourcentages massiques, sont reproduits ci-après.
Phase A

| | |
|---|---|
| Bis-Dioctadécylamide Dimer Dilinoleic | |
| Acid/Ethylènediamine Copolymer | 21 |
| Ethyl hexyl Hyd roxystéa rate | 11 |
| Polyisobutène hydrogéné | 56 |

Phase B

| | |
|---|---|
| Copolymère styrène hydrogéné /méthylstyrène/indène | 10 |
| Alcool Cétylique | 3,2 |
| Dibutyl Lauroyl Glutamide | 0,4 |
| Dibutyl Ethylhexanoyl Glutamide | 0,4 |

On prépare la composition (2) colorée selon le même procédé que celui décrit à l'Exemple 1. Les ingrédients qui la composent, ainsi que leurs proportions exprimées en pourcentages massiques, sont reproduits ci-après.

| | |
|---|---|
| Polyéthylène, cire d'abeille et cire d'ozokérite | 10,2 |
| Bentone, C₁₀-C₃₀ esters | 25,5 |
| Polyisobutène, esters, polydécène et octyldodécanol | 60,3 |
| Pigments et nacres | 0,7 |
| Conservateurs, agents actifs ou parfums | qsp 100 |

On mesure la dureté et le point de goutte des deux compositions par les méthodes utilisées dans l'Exemple 1. La composition (1) a une de dureté 6,4 N et un point de goutte égal à 82,8°C. La composition (2) a une de dureté 18,3 N et un point de fluidification égal à 75,4°C.

### Procédé de coulage

Dans un premier temps, on réalise au moins un dépôt de forme cylindrique de la composition transparente sur le support.

Dans un deuxième temps, on coule successivement des tranches cylindriques qui se superposent et qui comprennent chacune des dépôts de la composition (1) transparente et de la composition colorée (2).

Dans un troisième temps, on réalise au moins un dépôt de la composition transparente jusqu'à former un cylindre de la hauteur souhaitée dans la continuité du cylindre obtenu à l'étape précédente.

Le produit obtenu est un baume à lèvres en forme de stick (Figure 2). La première composition transparente laisse entrevoir la deuxième composition colorée qui forme par exemple un ruban de Moebius. L'utilisation du produit permet de déposer sur les lèvres un film à la fois brillant, grâce à la composition (1) et hydratant, grâce à la composition (2).

### EXEMPLE 3: Fond de teint multi-matière

### Préparation d'une composition

On prépare une composition selon le même procédé que celui décrit à l'Exemple 1. Les ingrédients qui la composent, ainsi que leurs proportions exprimées en pourcentages massiques, sont reproduits ci-après.

| | |
|---|---|
| Cire de polyéthylène | 3 |
| Cire d'abeille | 3 |
| Glycérides hydrogénés | 3,7 |
| Hectorite | 0,8 |
| Esters | 5,45 |
| Huiles | 9,7 |
| Filtres solaires liquides | 7,5 |
| Pigments | 19 |
| Eau | qsp 100 |
| Emollients et conservateurs | 5,6 |
| Vinyl diméthicone/méthicone silsesquioxane crosspolymer cellulose gum et polyméthylsilsesquioxane | 4,6 |
| Filtres solaires solides | 1,5 |

On mesure la dureté et le point de goutte des deux compositions par les méthodes utilisés dans l'Exemple 1. La composition a une dureté 12,8 N et un point de goutte égal à 58,4°C.

### Procédé de coulage

On réalise plusieurs dépôts successifs de la composition de fond de teint selon le procédé de l'invention, en utilisant comme support un objet en trois dimensions. Les dépôts sont réalisés de manière à recouvrir la surface du support en totalité sur une épaisseur constante, de manière à ce que le support soit enrobé et non visible.

Le produit obtenu est un fond de teint à effet frais et aqueux. La composition cosmétique est soutenue par le support et présente la même forme. La composition cosmétique peut être prélevée avec un pinceau. Au fur et à mesure des prélèvements de la composition, le support apparaît à l'utilisateur. Le support peut être en polymère thermoplastique et avoir été façonné selon un procédé de coulage par dépôts successifs, ou par moulage.

## Revendications

1. Procédé de fabrication d'un produit cosmétique par coulage lequel procédé comprend la succession d'étapes suivantes :
a) la fluidification d'au moins une composition contenant une huile, dotée d'un point de fluidification compris entre 30°C et 150°C, et d'une dureté moyenne - mesurée à 25°C à l'aide d'un texturomètre muni d'une sonde hémisphérique - comprise entre 0,5 N et 80 N, en la chauffant à une température de fluidification supérieure à son point de fluidification,
b) l'introduction de la composition fluidifiée dans une buse de coulage,
c1) le coulage par éjection de la composition fluidifiée à travers un orifice de la buse pour obtenir un volume, lequel volume est déposé sur un support et forme après refroidissement à 25°C un premier dépôt de ladite composition sur le support,
c2) la mise en mouvement relative - dans au moins une des trois directions de l'espace - de la buse par rapport audit support, le coulage de la composition fluidifiée à l'étape c1) et la mise en mouvement relative de la buse à l'étape c2) étant concomitants, de manière à former un premier dépôt de forme cylindrique, et la forme du volume éjecté étant identique à la forme du dépôt solide, en l'absence de contrainte mécanique ou thermique appliquée sur le dépôt après coulage,
d) l'arrêt du coulage, puis éventuellement
e) le déplacement relatif de la buse par rapport au support, et
f) la répétition des étapes a) à e) au moins une fois pour former au moins un autre dépôt sur ledit support, en utilisant la même composition ou une autre composition qui contient une huile, qui est dotée d'un point de fluidification compris entre 30°C et 150°C, et qui a une dureté moyenne - mesurée à 25°C à l'aide d'un texturomètre muni d'une sonde hémisphérique - comprise entre 0,5 N et 80 N.

2. Procédé selon la revendication 1, **caractérisé en ce que** la buse est dotée en partie inférieure d'un orifice dont une de ses dimensions va de 0,01 mm à 5 mm.

3. Procédé selon la revendication 1 ou 2, **caractérisé en ce que** la température de coulage est inférieure ou égale à la température de fluidification.

4. Procédé selon l'une des revendications 1 à 3, **caractérisé en ce que** le support ne sert pas de contenant au volume de composition fluidifiée pendant son refroidissement, si bien que le dépôt solide tient sa forme essentiellement de l'orifice de la buse.

5. Procédé selon l'une des revendications précédentes, **caractérisé en ce que** le diamètre moyen du dépôt est égal au diamètre de l'orifice de la buse.

6. Procédé selon l'une des revendications précédentes **caractérisé en ce que** le débit de coulage est compris entre 0,05 mm³/s et 2 mm³/s.

7. Procédé selon l'une des revendications précédentes **caractérisé en ce que** la viscosité dynamique de la composition à la température de coulage et à pression atmosphérique est comprise entre 1 mPa.s et 10 000 mPa.s, par exemple entre 100 mPa.s et 500 mPa.s.

8. Procédé selon l'une des revendications précédentes **caractérisé en ce que** la température de fluidification est supérieure de 1°C à 15°C au point de fluidification de la composition.

9. Procédé selon l'une des revendications précédentes **caractérisé en ce que** la composition est dotée d'un point de goutte supérieur à 50°C, par exemple compris entre 70°C et 85°C.

10. Procédé selon l'une des revendications précédentes, **caractérisé en ce que** le support est de nature cosmétique ou non cosmétique.

11. Procédé selon l'une des revendications précédentes, **caractérisé en ce qu'**il comprend une étape g) de coulage d'une matière thermoplastique réalisée antérieurement, postérieurement ou de façon simultanée à l'étape c).

## Patentansprüche

1. Verfahren zur Herstellung eines Kosmetikprodukts durch Gießen, wobei das Verfahren die folgende Schrittfolge umfasst:
a) die Verflüssigung von zumindest einer Zusammensetzung, die ein Öl enthält, das einen Verflüssigungspunkt aufweist, der zwischen 30 °C und 150 °C beträgt, und eine mittlere Härte - gemessen bei 25 °C mit Hilfe eines Texturmessgeräts, das mit einer Halbkugelsonde ausgestattet ist - die zwischen 0,5 N und 80 N beträgt, indem sie auf eine Verflüssigungstemperatur erwärmt wird, die höher als ihr Verflüssigungspunkt ist,
b) die Einführung der verflüssigten Zusammensetzung in eine Gussdüse,
c1) das Spritzgießen der verflüssigten Zusammensetzung durch eine Öffnung der Düse, um ein Volumen zu erhalten, wobei das Volumen auf einem Träger abgeschieden wird und nach Abkühlen auf 25 °C eine erste Ablagerung der Zusammensetzung auf dem Träger bildet,
c2) das relative Inbewegungsetzen - in zumindest einer der drei Raumrichtungen - der Düse in Bezug auf den Träger, wobei das Gießen der verflüssigten Zusammensetzung in Schritt c1) und das relative Inbewegungsetzen der Düse in Schritt c2) gleichzeitig erfolgen, um eine erste Ablagerung von zylindrischer Form zu bilden, und wobei die Form des ausgestoßenen Volumens identisch mit der Form der festen Ablagerung ist, in Abwesenheit von mechanischer oder thermischer Einschränkung, die nach dem Gießen auf die Ablagerung aufgebracht wird,
d) das Anhalten des Gießens, dann gegebenenfalls
e) die relative Verschiebung der Düse in Bezug auf den Träger, und
f) die zumindest einmalige Wiederholung der Schritte a) bis e), um zumindest eine weitere Ablagerung auf dem Träger zu bilden, indem die gleiche Zusammensetzung oder eine andere Zusammensetzung verwendet wird, die ein Öl enthält, das einen Verflüssigungspunkt aufweist, der zwischen 30 °C und 150 °C beträgt, und eine mittlere Härte - gemessen bei 25 °C mit Hilfe eines Texturmessgeräts, das mit einer Halbkugelsonde ausgestattet ist - die zwischen 0,5 N und 80 N beträgt.

2. Verfahren nach Anspruch 1, **dadurch gekennzeichnet, dass** die Düse im unteren Teil mit einer Öffnung versehen ist, wobei eine ihrer Abmessungen von 0,01 mm bis 5 mm reicht.

3. Verfahren nach Anspruch 1 oder 2, **dadurch gekennzeichnet, dass** die Gusstemperatur kleiner als die oder gleich der Verflüssigungstemperatur ist.

4. Verfahren nach einem der Ansprüche 1 bis 3, **dadurch gekennzeichnet, dass** der Träger nicht als Behältnis des Volumens der verflüssigten Zusammensetzung während ihrer Abkühlung dient, sodass die feste Ablagerung im Wesentlichen die Form der Öffnung der Düse annimmt.

5. Verfahren nach einem der vorhergehenden Ansprüche, **dadurch gekennzeichnet, dass** der mittlere Durchmesser der Ablagerung gleich dem Durchmesser der Öffnung der Düse ist.

6. Verfahren nach einem der vorhergehenden Ansprüche, **dadurch gekennzeichnet, dass** der Gussdurchsatz zwischen 0,05 mm³/s und 2 mm³/s beträgt.

7. Verfahren nach einem der vorhergehenden Ansprüche, **dadurch gekennzeichnet, dass** die dynamische Viskosität der Zusammensetzung bei der Gusstemperatur und bei atmosphärischem Druck zwischen 1 mPa.s und 10.000 mPa.s beträgt, zum Beispiel zwischen 100 mPa.s und 500 mPa.s.

8. Verfahren nach einem der vorhergehenden Ansprüche, **dadurch gekennzeichnet, dass** die Verflüssigungstemperatur höher als 1 °C bis 15 °C an dem Punkt der Verflüssigung der Zusammensetzung ist.

9. Verfahren nach einem der vorhergehenden Ansprüche, **dadurch gekennzeichnet, dass** die Zusammensetzung einen Tropfpunkt aufweist, der höher als 50 °C ist, zum Beispiel zwischen 70 °C und 85 °C beträgt.

10. Verfahren nach einem der vorhergehenden Ansprüche, **dadurch gekennzeichnet, dass** der Träger von kosmetischer oder nichtkosmetischer Natur ist.

11. Verfahren nach einem der vorhergehenden Ansprüche, **dadurch gekennzeichnet, dass** es einen Schritt g) des Gießens auf eine thermoplastische Weise umfasst, der vor, nach oder gleichzeitig mit Schritt c) umgesetzt wird.

## Claims

1. A casting method of manufacturing a cosmetic product, which process comprises the following succession of steps:
a) fluidization of at least one composition containing an oil, having a fluidization point between 30 ° C and 150 ° C, and an average hardness - measured at 25 ° C using a texturometer fitted with a hemispherical probe - between 0.5 N and 80 N, by heating it to a fluidization temperature above its fluidization point,
b) introduction of the fluidized composition into a pouring nozzle,
c1) casting by ejection of the fluidized composition through an orifice of the nozzle to obtain a volume, which volume is deposited on a support and, after cooling to 25 ° C, forms a first deposit of said composition on the support,
c2) the relative setting in motion of the nozzle with respect to said support - in at least one of the three spatial directions - the pouring of the fluidized composition in step c1) and the relative setting in motion of the nozzle to step c2) being concomitant, so as to form a first deposit having a cylindrical shape, and the shape of the ejected volume being identical to the shape of the solid deposit, in the absence of mechanical or thermal stress applied to the deposit after casting,
d) stopping the pouring, then possibly
e) the relative displacement of the nozzle with respect to the support, and
f) repeating steps a) to e) at least once to form at least one further deposit on said support, using the same composition or another composition which contains an oil, which has a fluidization point between 30 ° C and 150 ° C, and which has an average hardness - measured at 25 ° C using a texturometer fitted with a hemispherical probe - of between 0.5 N and 80 N.

2. Method according to claim 1, **characterized in that** the nozzle is provided, in the lower part, with an orifice, one of dimensions thereof ranging from 0.01 mm to 5 mm.

3. Method according to claim 1 or 2, **characterized in that** the casting temperature is less than or equal to the fluidization temperature.

4. Method according to one of claims 1 to 3, **characterized in that** the support does not serve as a container for the volume of fluidized composition during its cooling, so that the solid deposit takes its shape essentially from the orifice of the nozzle.

5. Method according to one of the preceding claims, **characterized in that** the average diameter of the deposit is equal to the orifice diameter of the nozzle.

6. Method according to one of the preceding claims, **characterized in that** the pouring rate is between 0.05 mm3 / s and 2 mm3 / s.

7. Method according to one of the preceding claims, **characterized in that** the dynamic viscosity of the composition at casting temperature and at atmospheric pressure is between 1 mPa.s and 10,000 mPa.s, for example between 100 mPa.s and 500 mPa.s.

8. Method according to one of the preceding claims, **characterized in that** the fluidization temperature is 1 ° C to 15 ° C above the fluidization point of the composition.

9. Method according to one of the preceding claims, **characterized in that** the composition has a dropping point greater than 50 ° C, for example between 70 ° C and 85 ° C.

10. Method according to one of the preceding claims, **characterized in that** the support is of cosmetic or non-cosmetic nature.

11. Method according to one of the preceding claims, **characterized in that** it comprises a step g) of casting a thermoplastic material carried out before, after or simultaneously with step c).
